# EUROPEAN PATENT APPLICATION

(11) **EP 2 805 683 A2**
(43) Date of publication of application: **26.11.2014**
(21) Application number: 14165506.8
(22) Date of filing: 22.04.2014
(51) Int. Cl.: A61B 18/14

(54) **Electrode assembly for catheter system**

(30) Priority: 21.05.2013 US 201361825793 P
(71) Applicant: St. Jude Medical, Cardiology Division, Inc., St. Paul MN 55117-9913 (US)
(72) Inventor: Raatikka, Amy Rochelle, Plymouth, MN Minnesota 55446 (US); Belk, Paul Aaron, Maple Grove, MN Minnesota 55331 (US); Cajamarca, Manuel Tobias, Plymouth, MN Minnesota 55446 (US)
(74) Representative: Smaggasgale, Gillian Helen

(57) **Abstract**

In an electrode assembly for a catheter system, a plurality of struts each extend from a proximal end to a distal end of the electrode assembly. Each strut spirals about the longitudinal axis of the electrode assembly and has a corresponding electrode disposed thereon. The electrode assembly is configurable between a collapsed configuration and an expanded configuration, with the electrodes being transversely spaced from the longitudinal axis of the electrode assembly a greater distance in the expanded configuration than in the collapsed configuration. In the expanded configuration the electrodes are transversely equally spaced from the longitudinal axis of the electrode assembly. In other aspects, at least one asymmetric element is provided on the electrode assembly to facilitate symmetric expansion about the longitudinal axis of the assembly. In another aspect, the electrode assembly is a single coil that winds about the longitudinal axis of the assembly.

## Description

### CROSS-REFERENCE TO RELATED APPLICATION

This application claims priority to provisional application serial No. 61/825,793 filed May 21, 2013, the entire specification of which is incorporated herein.

### BACKGROUND OF THE DISCLOSURE

### A. Field of the disclosure

The present disclosure relates generally to a catheter system for use in a human body, and more particularly to a multi-electrode catheter system, and even more particularly to an electrode assembly for a multi-electrode catheter system.

### B. Background Art

Catheter systems are well known in the art for use in medical procedures, such as diagnostic, therapeutic and ablative procedures. Typical catheter systems generally include an elongate catheter extending from a handle. A physician manipulates the catheter through the patient's vasculature to an intended site within the patient. The catheter typically carries one or more working components, such as electrodes or other diagnostic, therapeutic or ablative devices for carrying out the procedures. One or more controls or actuators may be provided on the handle for selectively adjusting one or more characteristics of the working components.

One particular example of a multi-electrode catheter system is an ablative catheter system in which the working component is a multi-electrode component carried at the distal end of a flexible catheter. A control wire extends within the catheter from the multi-electrode component to the handle to operatively connect the multi-electrode component to an actuator on the handle. Manipulating the actuator acts on the control wire to configure the multi-electrode component into a desired configuration for carrying out the ablative procedure. For example, in one such ablative catheter system made by St. Jude Medical, Inc. under the trade name EnligHTN, the multi-electrode component is an electrode assembly in the general form of a basket. Upon locating the electrode basket at a desired location within the patient, manipulating the actuator associated with the handle pulls on the control wire to reconfigure the electrode basket from a collapsed configuration to an expanded configuration in which the electrodes are intended to be in apposition with a surface, such as an arterial wall of the patient. It is thus desirable to facilitate apposition of as many of the electrodes of the electrode basket as possible against the arterial wall of the patient when the electrode basket is expanded to achieve optimal performance of the multi-electrode catheter system.

### BRIEF SUMMARY OF THE DISCLOSURE

In one embodiment, a catheter electrode assembly for an electrode catheter system generally comprises a plurality of struts each extending from a proximal end to a distal end of the electrode assembly. Each strut at least in part spirals about the longitudinal axis of the electrode assembly intermediate the proximal end and the distal end of the electrode assembly and has a corresponding electrode disposed thereon intermediate the proximal and distal ends of the electrode assembly. The electrode assembly is configurable between a collapsed configuration and an expanded configuration, with the electrodes being transversely spaced from the longitudinal axis of the electrode assembly a greater distance in the expanded configuration than in the collapsed configuration. In the expanded configuration the electrodes are transversely equally spaced from the longitudinal axis of the electrode assembly.

In another embodiment, an electrode assembly for an electrode catheter system generally comprises a plurality of struts each extending from a proximal end to a distal end of the electrode assembly. Each strut at least in part spirals about the longitudinal axis of the electrode assembly intermediate the proximal end and the distal end of the electrode assembly and has a corresponding electrode disposed thereon intermediate the proximal and distal ends of the electrode assembly. The electrode assembly is configurable between a collapsed configuration and an expanded configuration, with each electrode being spaced transversely from the longitudinal axis of the electrode assembly a greater distance in the expanded configuration than in the collapsed configuration. The electrode assembly includes at least one asymmetric element configured to facilitate a symmetrical expansion of the electrode assembly about the longitudinal axis thereof from the collapsed configuration to the expanded configuration of the electrode assembly

In yet another embodiment, an electrode assembly for an electrode catheter system generally comprises a plurality of struts each extending from a proximal end to a distal end of the electrode assembly. Each strut has a riser element disposed thereon intermediate the proximal end and the distal end of the electrode assembly. The electrode assembly is configurable between a collapsed configuration and an expanded configuration. In the expanded configuration the riser element of each strut extends transversely outward from the longitudinal axis of the electrode assembly a greater distance than any other point along the respective strut. Each strut further has an electrode disposed on the respective riser element

In still another embodiment, an electrode assembly for an electrode catheter system generally comprises a unitary coil having a plurality of winds and extending continuously from a proximal end to a distal end of the electrode assembly. A plurality of electrodes are disposed on the coil, with each electrode being disposed on a different one of the winds of the coil. The coil is configurable between a collapsed configuration and an expanded configuration, with the winds having a radius wherein the radius of the winds increases upon configuration of the coil from the collapsed configuration to the expanded configuration.

The foregoing and other aspects, features, details, utilities and advantages of the present disclosure will be apparent from reading the following description and claims, and from reviewing the accompanying drawings.

### BRIEF DESCRIPTION OF THE DRAWINGS

Figure 1 is a perspective view of one embodiment of a catheter system including a handle, a catheter and an electrode assembly having multiple electrodes, with the electrode assembly being in a collapsed configuration.
Figure 2 is a side elevation of the catheter system of Figure 1, with the electrode assembly being in an expanded configuration resulting from rotation of a rotatable actuator.
Figure 3 is a perspective view of the electrode assembly of Figure 1 with a plurality of struts carrying the multiple electrodes, the electrode assembly being in its collapsed configuration.
Figure 4 is a perspective view of the electrode assembly similar to Figure 3 but illustrating the electrode assembly in its expanded configuration.
Figure 5 is an enlarged perspective view of a distal end of the electrode assembly of Figure 3.
Figure 6 is an enlarged perspective view of a proximal end of the electrode assembly of Figure 3.
Figure 7 is a schematic view of the electrode assembly at one stage of manufacturing thereof at which the electrode assembly is in the form of a tube with portions of the tube cut away to form struts of the electrode assembly.
Figure 8 is a schematic view of the electrode assembly at the stage of manufacturing illustrated in Figure 7, with the tube being in a longitudinally opened and laid flat orientation for illustrative purposes.
Figure 9 is a schematic view of the electrode assembly similar to Figure 7 but with the electrode assembly at a subsequent stage of manufacturing in which the struts are heat set in what later becomes the collapsed configuration of the electrode assembly.
Figure 10 is a schematic view of another embodiment of an electrode assembly at one stage of manufacturing thereof with the electrode assembly being in a longitudinally opened and laid flat orientation for illustrative purposes.
Figure 11 is a schematic view of another embodiment of an electrode assembly at one stage of manufacturing thereof with the electrode assembly being in a longitudinally opened and laid flat orientation for illustrative purposes.
Figure 12 is a schematic view of another embodiment of an electrode assembly at one stage of manufacturing thereof with the electrode assembly being in a longitudinally opened and laid flat orientation for illustrative purposes.
Figure 13 is a schematic view of another embodiment of an electrode assembly at one stage of manufacturing thereof with the electrode assembly being in a longitudinally opened and laid flat orientation for illustrative purposes.
Figure 14 is a schematic view of another embodiment of an electrode assembly at one stage of manufacturing thereof with the electrode assembly being in a longitudinally opened and laid flat orientation for illustrative purposes.
Figure 15 is a schematic view of another embodiment of an electrode assembly at one stage of manufacturing thereof with the electrode assembly being in a longitudinally opened and laid flat orientation for illustrative purposes.
Figure 16 is a schematic view of another embodiment of an electrode assembly at one stage of manufacturing thereof with the electrode assembly being in a longitudinally opened and laid flat orientation for illustrative purposes.
Figure 17 is a schematic perspective view of another embodiment of an electrode assembly suitable for use with the catheter system of Figure 1.
Figure 18 is a schematic perspective view of another embodiment of an electrode assembly suitable for use with the catheter system of Figure 1.
Corresponding reference characters indicate corresponding parts throughout the several views of the drawings.

### DETAILED DESCRIPTION OF THE DISCLOSURE

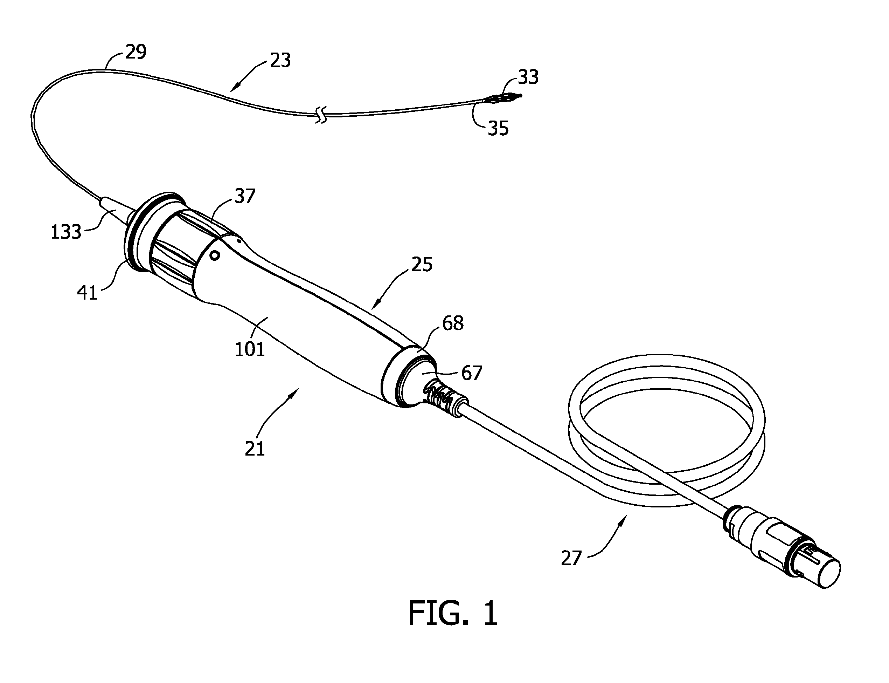

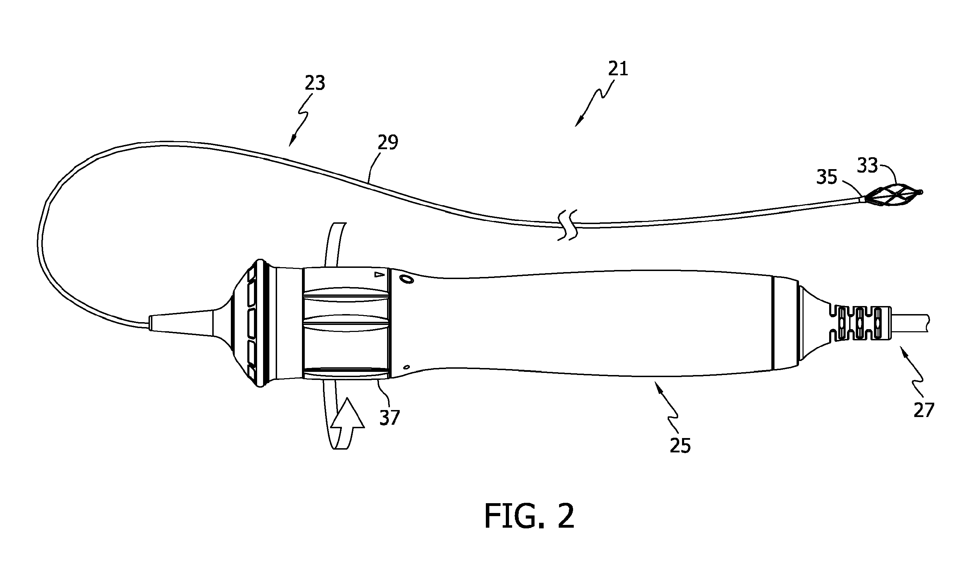

Referring now to the drawings, and in particular to Figures 1 and 2, one embodiment of a catheter system 21 includes a flexible catheter 23, a handle 25 to which the catheter is connected, and a conductor assembly 27 for electrically connecting the catheter system to a suitable power supply (not shown). As one example, the catheter system 21 illustrated and described herein is suitably constructed for use as an ablation system, such as a renal or heart ablation system. More particularly, the illustrated catheter system 21 is a multi-electrode renal denervation system. One example of such a catheter system 21 is currently made by St. Jude Medical, Inc. under the trade name EnligHTN. General operation of a multi-electrode renal denervation system is known to those of skill in the art and is not described further herein except to the extent necessary to describe the present embodiments. It is also understood that the catheter system 21 may be used for any other suitable treatment or purpose without departing from the scope of this disclosure. Additionally, while the catheter system 21 is illustrated and described herein as including a flexible catheter 23, the system may further include other components used, for example, to guide the flexible catheter into the patient - such as, without limitation, a relatively more rigid guide catheter (not shown).

The catheter 23 includes an elongate, flexible hollow shaft 29 connected to the handle 25 at or near a proximal or rear end of the catheter shaft (not shown because it is hidden by a connector at the front end of the handle 25), and an electrode assembly 33 disposed at or near a distal or front end 35 of the catheter shaft. It is understood, however, that the electrode assembly 33 may be disposed anywhere along the catheter shaft 29 intermediate the proximal end and the distal end 35 thereof without departing from the scope of this disclosure. As used herein, the terms proximal and front, and distal and rear, are used with reference to the orientation of the catheter system 21 illustrated in the various drawings and for the purpose of describing the various embodiments set forth herein, and are not intended as limiting the catheter system and related components to having any particular orientation upon assembly or during operation thereof. In particular, the terms proximal and rear refer to a longitudinal position that is relatively nearer to the handle 25 while the terms distal and front refer to a longitudinal position that is relatively farther from the handle.

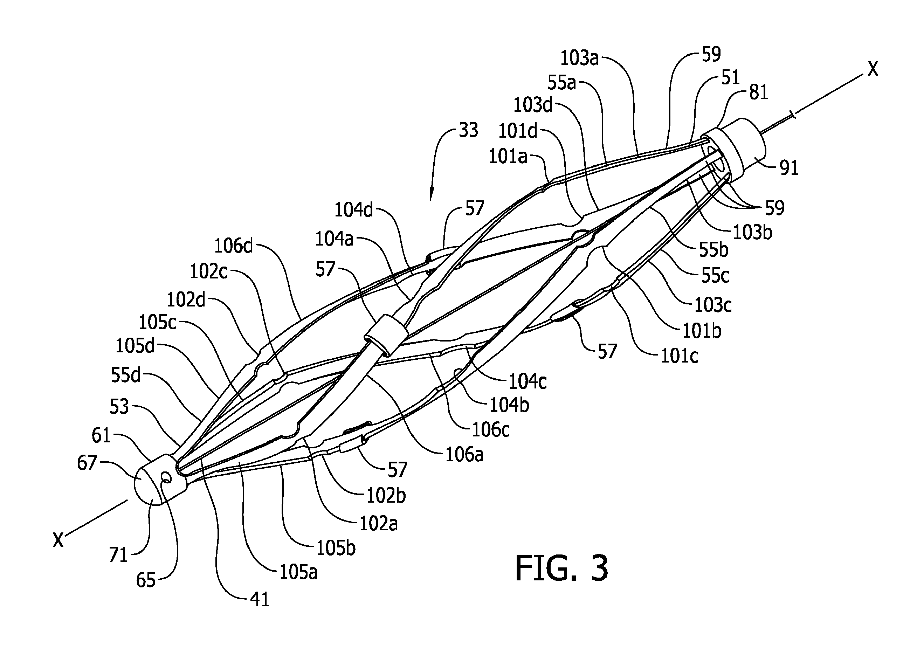

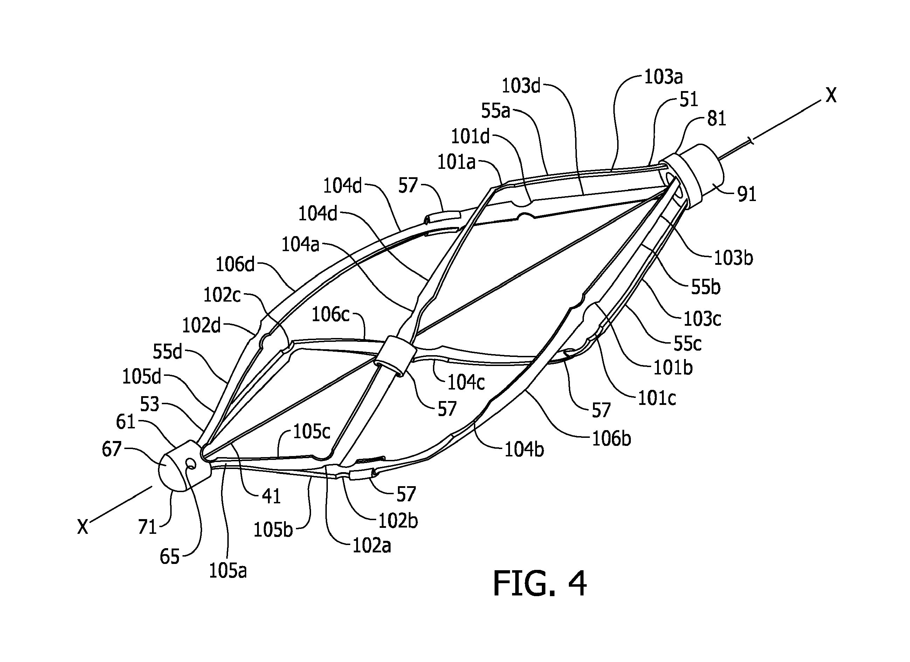

The illustrated electrode assembly 33 is in the form of what may be referred to as an electrode basket and is suitably configurable between a collapsed configuration (Figures 1 and 3) for maneuvering and positioning the electrode assembly in the patient, and an expanded configuration (Figures 2 and 4) for operation of the electrode assembly to perform a desired procedure such as an ablation procedure. An annular (e.g., ring-shaped) actuator 37 is mounted on the handle 25 for rotation relative thereto and is operatively connected to the electrode assembly 33 for selectively configuring the electrode assembly between its collapsed and expanded configurations. It is understood that another suitable actuator (e.g., slide, push button, lever, etc.) may be used instead of the rotating actuator 37 to selectively configure the electrode assembly 33 without departing from the scope of this disclosure. In some embodiments, the electrode assembly 33 may be selectively adjustable between an infinite number of configurations (e.g., degrees of expansion) between its collapsed and expanded configurations using the actuator 37.

A control line, such as a suitable cable or pull wire 41 (Fig. 3) extends from the electrode assembly 33 within the hollow catheter shaft 29 and into the handle 25 for operative connection with the actuator to thereby operatively connect the actuator 37 with the electrode assembly. In some embodiments two or more pull wires, cables or other suitable control lines may be used for selectively configuring the electrode assembly 33. It is also understood that the control line 41 may be any suitable control line other than a pull wire, such as a cable, string, tie, compression member or other suitable control to operatively connect the electrode assembly 33 to the actuator 37. In other embodiments, any suitable conventional manner for actuating the otherwise selectively configuring the electrode assembly 33 may be used. A suitable twisted electrical wire bundle (not shown) also extends through the hollow catheter shaft 29 from the handle to the electrode assembly to deliver power to the electrode assembly.

With reference now to Figure 3, the electrode assembly 33 has a proximal end 51 at which the assembly is connected to the catheter shaft 29 (e.g., to the distal end 35 of the catheter shaft in the embodiment of Figures 1 and 2), a distal end 53 that in the illustrated embodiment also defines a distal end, or tip, of the catheter 23, and a longitudinal axis X. The illustrated electrode assembly 33 comprises a set of four struts 55a-d, extending coextensively with each other from the proximal end 51 to the distal end 53 of the electrode assembly in circumferentially equal spaced relationship with each other about the longitudinal axis X of the electrode assembly. In other embodiments, the electrode assembly 33 may comprise more or less than four struts 55a-d without departing from the scope of this disclosure. It is also contemplated that the struts 55a-d may be other than equally spaced from each other circumferentially, and/or the struts may be other than coextensive with each other, and remain within the scope of this disclosure.

Each of the struts 55a-d carries at least one electrode 57 disposed at a respective longitudinal position along the strut, i.e., at a respective longitudinal distance along the longitudinal axis X from the proximal end of the electrode assembly. In the embodiment of Figure 3, each of the electrodes 57 is at a different longitudinal position. It is understood that in other embodiments the electrodes 57 may be at longitudinal positions other than those shown in Figure 3. It is also understood that two, three or all of the electrodes 57 may instead be at the same longitudinal position. It is also understood that multiple electrodes 57 may be carried by any one or all of the struts 55a-d, e.g., with the electrodes on any given strut spaced longitudinally from each other along the strut. While not shown in Figure 3, a respective suitable sheathing or sleeve, constructed of a polymeric material, circumferentially encloses each of the struts 55a-d along their respective lengths. The segment of the control line 41 that extends from the proximal end to the distal end of the electrode assembly may likewise be circumferentially enclosed by a suitable polymeric sheathing or sleeve.

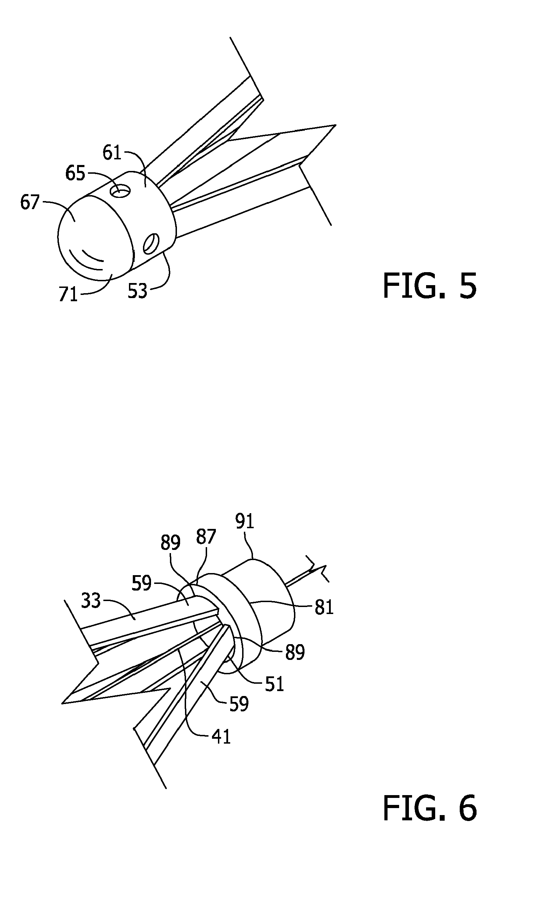

At the distal end 53 of the electrode assembly 33, the struts 55a-d terminate at, and in one embodiment for making the electrode assembly are formed integrally with, a connecting ring 61 (as best illustrated in Figure 5) having a central opening that is coaxial with the longitudinal axis X of the electrode assembly. In the illustrated embodiment, multiple holes 65 are formed in the sidewall of the connecting ring 61 in spaced relationship with each other about the circumference of the connecting ring and are open to the central opening of the connector. In other embodiments, however, the holes 65 may be omitted. Suitable polymeric sheathing (not shown) may surround the connecting ring 61 to cover the holes 65 following assembly of the electrode assembly 33. As seen in Figures 3 and 4, a blunt tip 67 includes a rounded head 71 having a cylindrical body (not shown) extending longitudinally therefrom and being generally hollow along its length such that the rounded head closes the distal end of the body.

The control line 41 extends generally along the longitudinal axis X of the electrode assembly 33 through the body of the tip 67 where it is secured to the tip by braising, adhesive, welding, soldering or other suitable securement technique. The tip body is sized in transverse cross-section, e.g., outer diameter, to be received through and seat within the central opening of the connecting ring 61 with the head 71 of the tip 67 abutting against the end of the connecting ring as seen in Figure 5. The holes 65 spaced about the circumference of the connecting ring 61 allow a suitable adhesive to be supplied through the holes for securing the tip 67 on the connecting ring - thereby connecting the distal end 53 of the electrode assembly 33 to the control line 41 for operative connection with the actuator 37 on the handle 25. In other embodiments the struts 55a-d may be retained at the distal end 53 of the electrode assembly 33 in another suitable manner and remain within the scope of this disclosure. It is also contemplated that the struts 55a-d and connecting ring 61 may be formed separate from each other and subsequently secured together by any suitable securement technique.

Referring to Figure 6, at the proximal end 51 of the electrode assembly 33, longitudinal end segments 59 of the struts 55a-d are connected to the catheter shaft 29 by a suitable bushing 81. The bushing 81 includes a tubular cylindrical body (not shown) through which the control line 41 extends from the catheter shaft 29 to the electrode assembly 33. An annular flange 87 extends radially outward from the longitudinally outer end of the bushing 81. The flange 87 has four slots 89 (corresponding to the respective longitudinal end segments 59 of the struts 55a-d) extending longitudinally therethrough radially outward of the cylindrical body of the bushing 81 and in circumferentially spaced relationship with each other. As illustrated in Figure 6, the longitudinal end segments 59 of the struts 55a-d extend through the respective slots 89 and along the outer surface of the cylindrical body of the bushing 81.

The body of the bushing 81 (along with the longitudinal end segments 59 of the struts 55a-d) is fitted with a polyimide sleeve 91 filled with suitable adhesive to secure the sleeve and longitudinal end segments of the struts to the bushing. The bushing 81, struts 55a-d and polyimide sleeve 91 are inserted into the distal end 35 of the hollow catheter shaft 29 and secured to the catheter shaft by suitable adhesive to secure the proximal end 51 of the electrode assembly 33 to the distal end of the catheter shaft. It is understood that the struts 55a-d may be connected to the catheter shaft 29 by any other suitable connection that allows the electrode assembly 33 to function in the manner described herein.

The electrode assembly 33 thus has a length defined by the distance along the longitudinal axis X from the proximal end 51 to the distal end 53 of the electrode assembly. To configure the electrode assembly 33 from its collapsed configuration (e.g., as illustrated in Figures 1 and 3) to its expanded configuration (e.g., as illustrated in Figures 2 and 4), rotation of the actuator 37 relative to the handle 25 operatively pulls on the control wire 41 to thereby pull the tip (i.e., the distal end 53) of the electrode assembly toward the proximal end 51 of the electrode assembly along the longitudinal axis X thereof. As the distance between the distal end 53 and the proximal end 51 of the electrode assembly 33 is shortened (i.e., as the length of the electrode assembly decreases), the struts 55a-d are longitudinally compressed and thus forced to bend, or flex transversely outward away from the longitudinal axis X of the electrode assembly to form the expanded configuration of the electrode assembly. As used herein, the expanded configuration of the electrode assembly refers to any transverse movement of the struts 55a-d outward from the collapsed (e.g., initial or pre-set) configuration of the electrode assembly, and may be variably adjusted. Accordingly, it is understood that in the expanded configuration the electrode assembly 33 may be expanded more or less than as illustrated in the various embodiments herein. It is also understood that the collapsed configuration is not intended to mean the most compressed form in which the electrode assembly 33 may be configured, but rather the relaxed configuration of the electrode assembly free from any external compression forces (such as when compressed to fit the electrode assembly into a guide tube or lumen).

With particular reference to Figures 3 and 4, the struts 55a-d of the illustrated electrode assembly 33 are suitably configured in both the collapsed and expanded configurations to generally spiral about the longitudinal axis X of the electrode assembly along at least a portion of the span of each respective strut intermediate the proximal and distal ends of the electrode assembly. It is believed that the spiral configuration of each of the struts 55a-d provides good radial (transverse) strength and stability to the electrode assembly, particularly in its expanded configuration, to thereby facilitate and maintain apposition of all of the electrodes against the arterial wall and provide resistance against deflection upon movement of the arterial wall, such as during a spasm. The spiral configuration also provides mechanical support to the arterial wall.

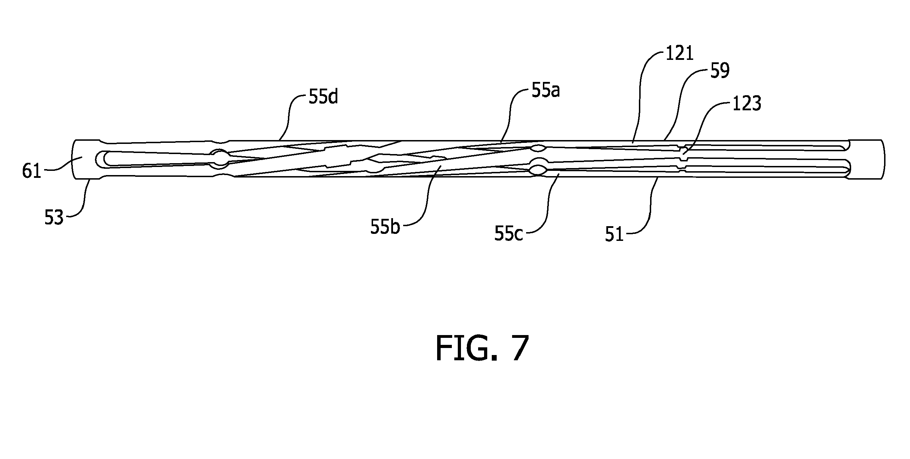

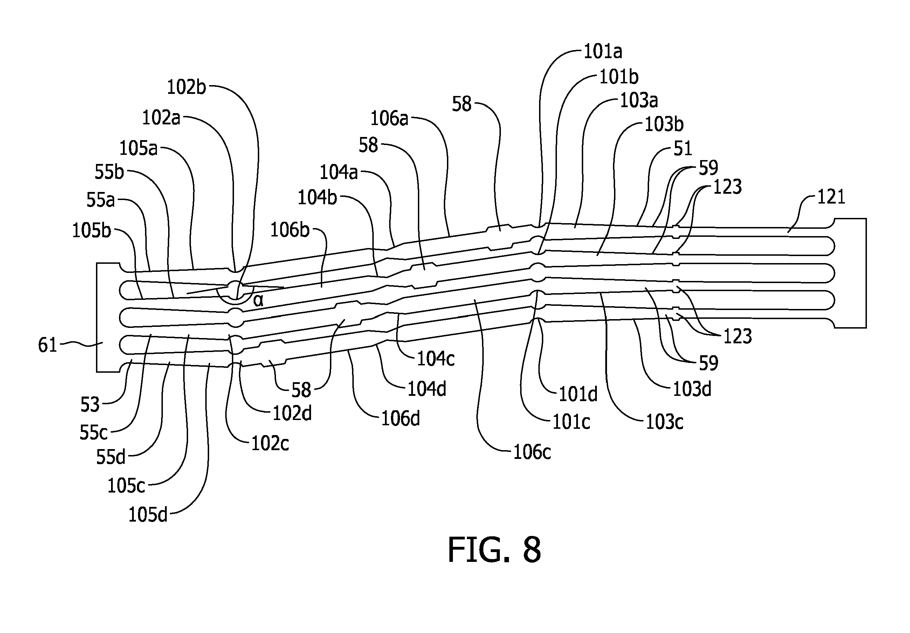

Figures 7 and 8 illustrate one embodiment of a method for making the electrode assembly 33 of Figures 1-6. A unitary tube 121 of a material having sufficient strength and shape memory characteristics, such as Nitinol™, is used. The material or materials from which the tube 121 is constructed, however, may be any other suitable material and remain with the scope of this disclosure. The desired pattern of struts 55a-d is then laser cut into the tube 121 so that the struts spiral about the longitudinal axis along at least a portion of the span of each respective strut from the proximal to the distal ends 51, 53 of the electrode assembly 33. In one suitable embodiment, the circumferential rotation angle of each strut 55a-d is about 90 degrees from the proximal end 51 to the distal end 53. In other embodiments the rotation angle of each spiraling strut 55a-d may be more or less than 90 degrees.

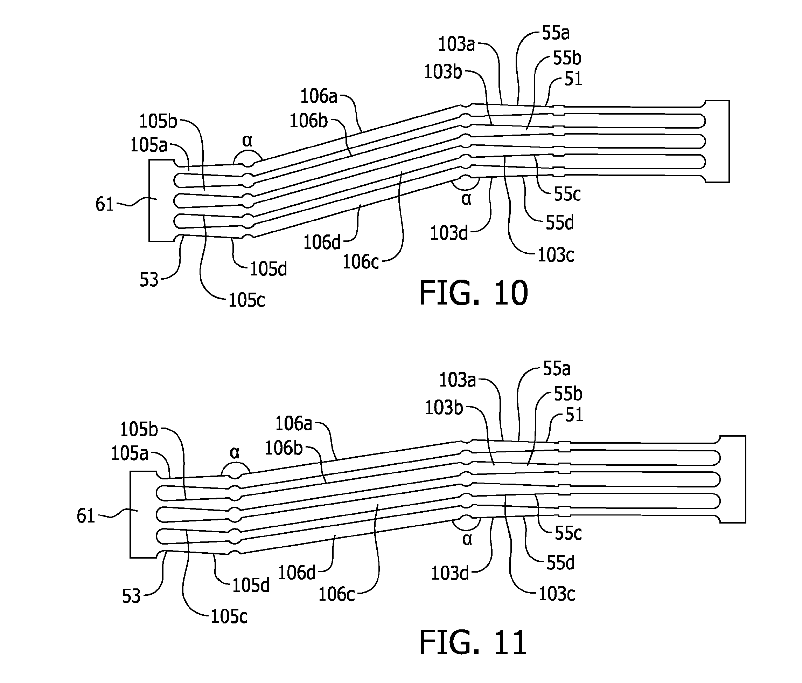

The tube 121 (e.g., as illustrated in Figure 7) is initially longer than the length of the finished electrode assembly 33 (as illustrated in Figure 3). The electrode assembly 33 illustrated in Figure 8 is representative of the tube 121 (although cut lengthwise and laid flat) of Figure 7 as initially formed. An alignment member 123 is formed on each strut 55a-d during the laser cutting process longitudinally outward of the ends of the struts near what eventually becomes the proximal end 51 of the electrode assembly 33. In the illustrated embodiment, each of the struts 55a-d includes a widened section defining an electrode pad 58 on which an electrode 57 (e.g., as illustrated in Figure 3) is mounted on the respective strut. It is understood that in other embodiments (e.g., as illustrated in Figures 10, 11 and 12) the struts 55a-d may not be configured to include an electrode pad 58, in which instance the electrode 57 is mounted on each strut at the desired longitudinal position of the electrode.

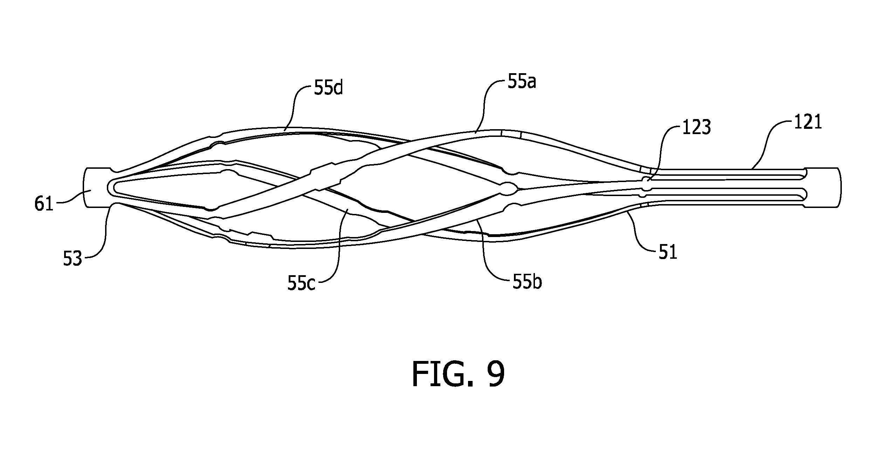

Once the struts 55a-d are formed in the tube 121, an initial slight amount of preset bend is formed in the tube 121 as illustrated in Figure 9 using an internal and external die assembly or other suitable technique and then heat setting the tube to give the tube shape its collapsed configuration. Such preset gives the struts 55a-d increased shaped memory to retain the spiral configuration thereof and to facilitate more predictable bending of the struts into the desired expanded configuration of the electrode assembly. Following the heat setting, the tube 121 is cut adjacent the alignment members 123 to define the longitudinal end segments 59 (Figure 6) of the struts 55a-d for connecting the struts to the bushing 81 and subsequently to the catheter shaft 29 in the manner described previously. The tip 67 is secured to the distal end 53 of the electrode assembly 33 (e.g., to the connecting ring 61) in the manner described previously.

With reference to Figure 8, to further facilitate predictable bending of the struts 55a-d, each strut includes a pair of hinges 101a-d, 102a-d in longitudinally spaced relationship with each other to delineate (for purposes of description herein) a pair of longitudinally opposite legs 103a-d, 105a-d interconnected by a central segment. In particular, with reference to the uppermost strut 55a in Figure 8, a longitudinally proximal leg 103a extends from the center of the one hinge 101a to the alignment member 123 and a longitudinally distal leg 105a extends from the center of the other hinge 102a to the connecting ring 61. In the illustrated embodiment, the proximal leg 103a-d and the distal leg 105a-d of each strut 55a-d are of generally equal length. In other embodiments, however, the proximal leg 103a-d and the distal leg 105a-d may be of unequal length. Also, in the illustrated embodiment each strut 55a-d has a proximal leg 103a-d, central segment 106a-d and distal leg 105a-d of lengths equal to the proximal leg, central segment and distal leg of each of the other struts so as to maintain symmetry of the electrode assembly 33. It is understood, though, that the respective lengths of the proximal leg 103a-d, center segment 106a-d and distal leg 105a-d of one strut may be different from that of one or more of the other struts. In the embodiment of Figure 8, each strut 55a-d has an additional hinge 104a-d or otherwise additional narrowing of the strut formed at the midsection of each center segment 106a-d to further facilitate predictable bending of the strut. However, this additional hinge 104a-d may be omitted in other embodiments.

To form the spiral configuration of each strut 55a-d, the center segment 106a-d of each strut (i.e., extending between the hinges 101 a-d, 102a-d) is angled relative to the longitudinally opposite legs 103a-d, 105a-d. For example, in one suitable embodiment an angle α in the range of about 165 degrees to about 175 degrees is defined by the center segment 106a-d and each of the legs 103a-d, 105a-d of each of the struts 55a-d. For example, in the embodiment of Figure 8 the angle α is about 172 degrees. In the embodiment illustrated in Figure 10 (discussed in further detail later herein), the angle α is about 165 degrees. The degree of the angle α determines the circumferential rotation angle of the strut 55a-d. While in the illustrated embodiment the angle between the center segment 106a-d and the proximal leg 103a-d is equal to the angle between the center segment and the distal leg 105a-d is the same, in other embodiments it is contemplated that the angle between the center segment and one leg may be different from the angle between the center segment and the other leg without departing from the scope of this disclosure.

Still referring to Figure 8, the proximal leg 103a-d of each strut has a width that decreases continuously (i.e., tapers, or narrows) from adjacent the hinge 101a-d to adjacent the alignment member 123. Likewise, the legs 105a-d each have a width that decreases continuously (i.e., tapers, or narrows) from adjacent the hinge 102a-d to adjacent the connecting ring 61. In other embodiments, the width of each proximal leg 103a-d and/or distal leg 105a-d may be uniform along its length, or it may be tapered in a manner other than as illustrated. The width of each center segment 106a-d of each strut 55a-d is generally uniform along its length, with the exception of the midsection hinge 104a-d if such hinges are present. Each strut 55a-d has a narrowed width intermediate the center segment 106a-d and the proximal leg 103a-d to define the hinge 101a-d and another narrowed width intermediate the center segment and the distal leg 105a-d to define the hinge 102a-d. In the illustrated embodiment the width of each strut 55a-d at the hinge 101a-d is equal to the width of the strut at the other hinge 102a-d. However, in other embodiments the width of the strut 55a-d at the hinge 101a-d may be different from the width of the strut at the other hinge 102a-d and remain within the scope of this disclosure.

The hinges 101a-d, 102a-d of the various embodiments herein are each formed by generally U-shaped cut-outs on opposite sides of each strut 55a-d so that the strut material is continuous across the narrowed width of the strut. The rounded contour of each of the cut-outs reduces the stress at the hinge 101a-d, 102a-d upon bending of the strut 55a-d. In other embodiments, the cut-outs may be other than U-shaped, such as V-shaped or other suitable shape and remain within the scope of this disclosure. It is also understood that one or both of the hinges 101 a-d, 102a-d may alternatively be formed by cutting an opening (not shown) between the side edges of the strut 55a-d at the respective hinge so that the narrowed width of the strut at the hinge is defined by the combined widths of the webs of strut material remaining on both sides of such an opening. It also understood that only one of the hinges, either 101 a-d or 102a-d may be used while the other is omitted.

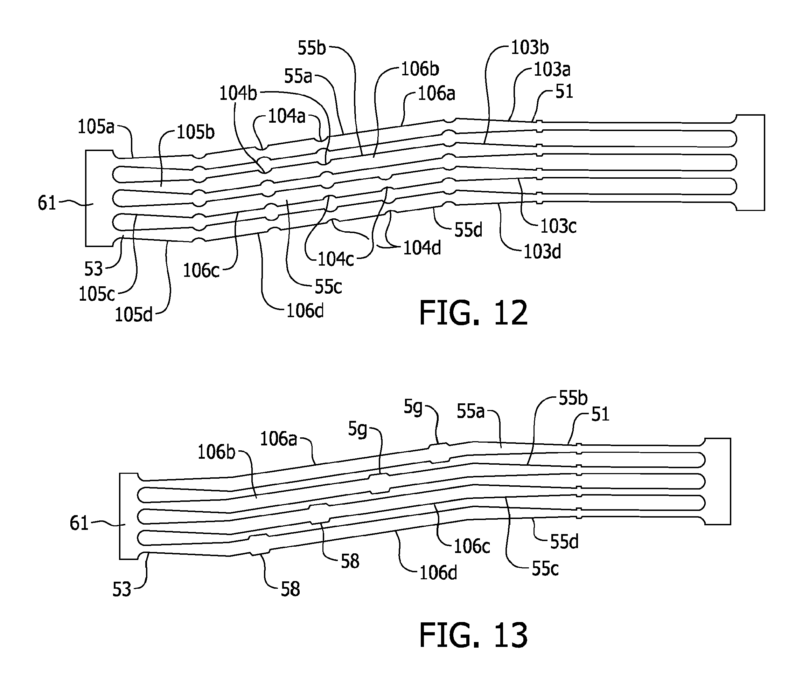

Figure 10 illustrates another embodiment similar to that of Figure 8, but with the electrode pads 58 omitted. In this embodiment, the angle α between the center segment 106a-d and each of the legs 103a-d, 105a-d is about 165 degrees. In the alternative embodiment of Figure 11, the angle α between the center segment 106a-d and each of the legs 103a-d, 105a-d is about 172 degrees. In the additional alternative embodiment of Figure 12, multiple hinges 104a-d are formed in each of the center segments 106a-d of each strut 55a-d to further facilitate bending of the struts upon configuration to the expanded configuration of the electrode assembly 33. It is understood that in other embodiments more than two hinges may be formed in each of the center segments.

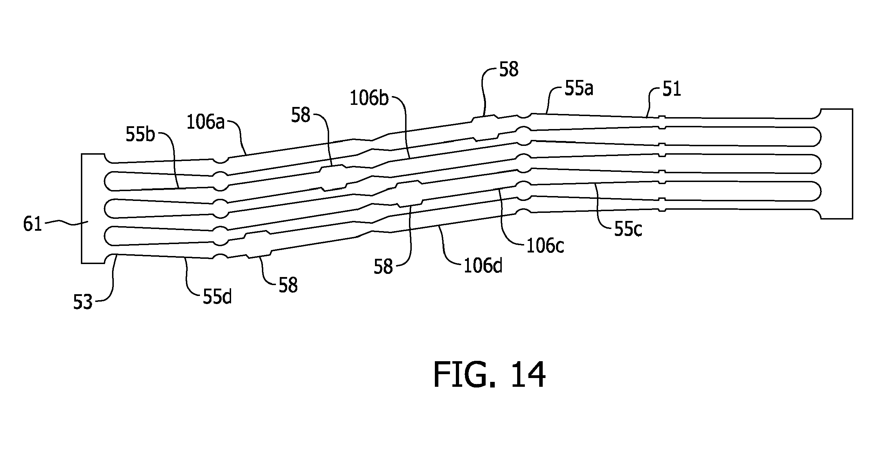

In the embodiment of Figure 13, the struts 55a-d are free of any hinges but are otherwise similar to the struts of the embodiment of Figure 8. As illustrated in this embodiment, the longitudinal positions of the electrode pads 58 are staggered sequentially along the length of the electrode assembly 33 - i.e., with the longitudinal position of the respective electrode pads 58 (and hence the electrodes 57) increasing sequentially from the first strut 55a to the fourth strut 55d about the circumference of the electrode assembly. This same electrode arrangement is present in the hinged embodiment of Figure 8 discussed previously herein. In the alternative embodiment of Figure 14, which has a hinge arrangement similar to the embodiment of Figure 8, all of the electrode pads 58 (and hence the electrodes 57) are at different longitudinal positions, but are not longitudinally positioned sequentially about the circumference of the electrode assembly 33. By positioning the electrodes 57 at different longitudinal positions on each of the struts 55a-d, the electrode assembly 33 is capable of circumferentially compressing down to a smaller cross-section beyond that of the preset collapsed configuration of Figure 3 to facilitate positioning of the electrode assembly in a guide tube or carrier. It is understood, however, that in some embodiments the longitudinal position of the electrode 57 on one of the struts 55a-d may be the same as the longitudinal position of the electrode of at least one of the other struts.

In each of the embodiments of Figures 8, 10, 11, 13 and 14, the struts 55a-d are configured to be generally symmetric from one strut to the next, including the strut dimensions, lengths and widths of the proximal legs, distal legs and center segments, locations of the hinges and the angle α that defines the amount of spiral. However, where the electrodes 57 (whether on a strut having an electrode pad 58 or a strut having no electrode pad) are at different longitudinal positions along the respective struts 55a-d, as illustrated in the embodiments of Figures 8, 13 and 14, the electrode assembly 33 may become asymmetric and, as a result, bending forces are applied to the struts asymmetrically upon configuring of the electrode assembly to its expanded configuration. Accordingly, in some embodiments, the struts 55a-d may be further configured to have one or more neighboring or distant asymmetric elements to balance out the asymmetry resulting from the different longitudinal positions of the electrodes or other assembly components.

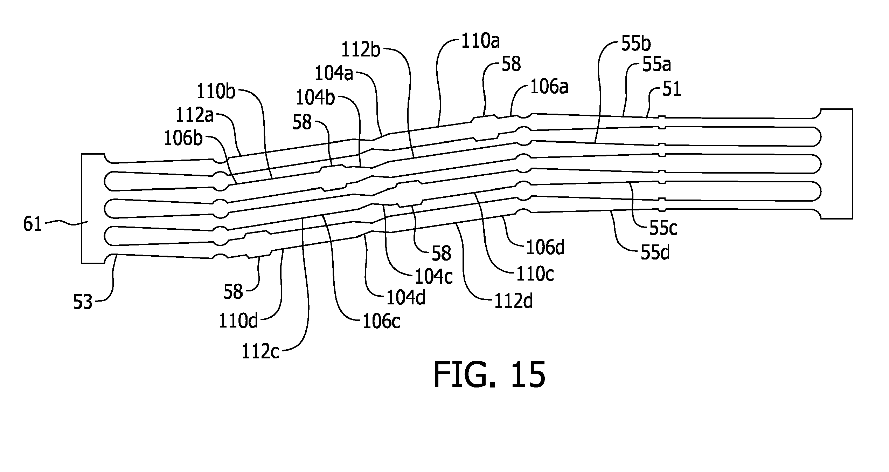

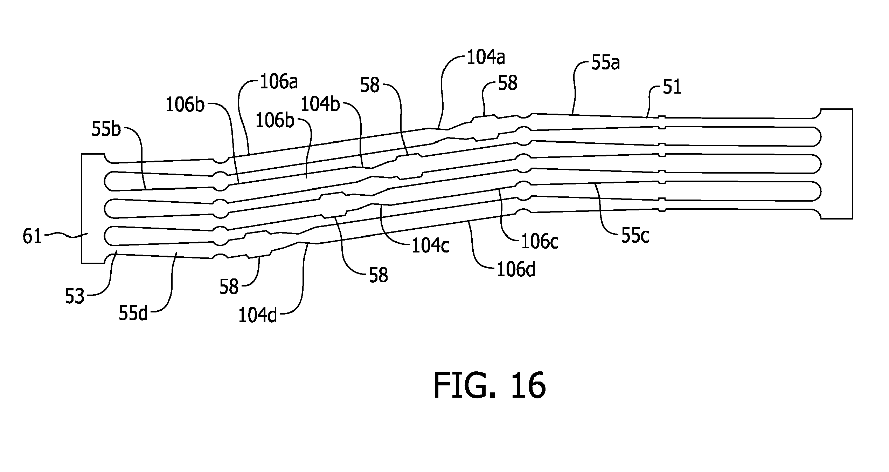

For example, in the embodiment illustrated in Figure 15, the electrode pads 58 (and hence the electrodes 57 - which are arranged in a pattern otherwise similar to that of the embodiment of Figure 14) are respectively disposed on each center segment 106a-d on one longitudinal side of the midsection hinge 104a-d so that each center segment defines an electrode side 110a-d and a non-electrode side 112a-d (as indicated by arrows for two of the struts 55a and 55c) of the center segment. To balance the asymmetry of the electrode locations, the non-electrode side 112 a-d of each center segment 106a-d has a greater width than the electrode side 110a-d of the respective center segment. In the alternative embodiment of Figure 16, the midsection hinges 104a-d are located generally immediately adjacent to the electrode locations. The midsection hinges 104a-d may be on either longitudinal side of the electrode pad 58 (or electrode 57) as illustrated in Figure 16, or they may be all on the same longitudinal side of the electrode. It is contemplated that a combination of the increased widths of the struts 55a-d and the relocated midsection hinges 104a-d may be used to balance the asymmetry. It is also understood that the struts 55a-d may include other suitable asymmetric elements to balance the asymmetry of the electrode assembly 33 without departing from the scope of this disclosure.

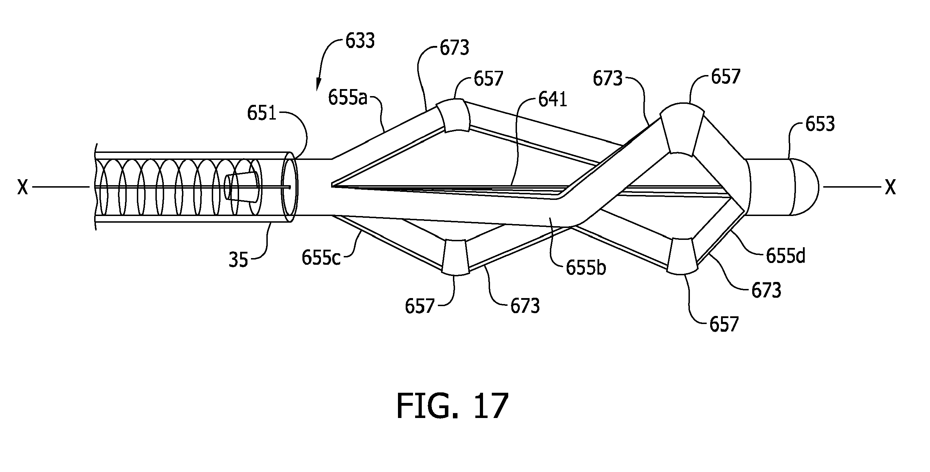

Figure 17 illustrates another embodiment of an electrode assembly 633 for use with the catheter system 21 illustrated in Figures 1 and 2. The electrode assembly 633 is configurable between a collapsed configuration for maneuvering and positioning the electrode assembly in the patient, and an expanded configuration (not shown) for operation of the electrode assembly to perform a desired procedure such as an ablation procedure. As seen in Figure 17, a control line, such as a suitable cable or pull wire 641, extends within the hollow catheter shaft 29 and into the handle 25 for operative connection with the actuator 37 to thereby operatively connect the actuator 37 with the electrode assembly.

The illustrated electrode assembly 633 comprises a set of four struts 655a-d extending from a proximal end 651 to a distal end 653 of the electrode assembly in circumferentially spaced relationship with each other about a longitudinal axis X of the electrode assembly. In other embodiments, the electrode assembly 633 may comprise more or less than four struts 655a-d without departing from the scope of this disclosure. Each of the struts 655a-d carries at least one electrode 657 disposed at a respective longitudinal position along the strut, i.e., at a respective longitudinal distance along the longitudinal axis X from the proximal end 651 of the electrode assembly 633. In the embodiment of Figure 17, the electrodes 657 are aligned in pair at generally the same longitudinal position. That is, the electrodes 657 on two opposed struts (e.g., 655a and 655c) are at a first longitudinal position and the electrodes on the other two opposed struts (e.g., 655b and 655d) are at a second longitudinal position. It is understood, however, that the electrodes 657 can be disposed at any suitable position along the respective strut 655a-d. It is also understood that multiple electrodes 657 may be carried by any one or all of the struts 655a-d, e.g., with the electrodes on any given strut spaced longitudinally from each other along the strut.

The electrode assembly 633 has a length defined by the extent of the control line 641 along the longitudinal axis X from the proximal end 651 to the distal end 653 of the electrode assembly. To configure the electrode assembly 633 from its collapsed configuration to its expanded configuration, rotation of the actuator 37 (as indicated by the arrow in Figure 2) relative to the handle 25 operatively pulls on the control wire 641 to thereby pull the distal end 653 of the electrode assembly toward the proximal end 651 of the electrode assembly along the longitudinal axis X thereof. As the distance between the distal end 653 and the proximal end 651 of the electrode assembly 633 is shortened (i.e., as the length of the electrode assembly decreases), the struts 655a-d are longitudinally compressed and thus forced to bend, or flex transversely outward away from the longitudinal axis X of the electrode assembly to form the expanded configuration of the electrode assembly.

In accordance with one embodiment, each of the struts 655a-d has a riser element 673 intermediate the proximal and distal ends 651, 653 of the electrode assembly 633 to facilitate a greater transversely outward point of contact of the electrodes against the arterial wall upon configuring the electrode assembly in its expanded configuration. In the illustrated embodiment of Figure 17, for example, the riser element comprises a transversely outward localized bend that is formed in the strut, such as by suitable heat setting as described previously herein. Upon expansion of the electrode assembly 633, the prior-formed bend (i.e., the riser element) 673 is maintained in the strut 655a-d so that the electrode, which is disposed at or adjacent the apex of the bend, is disposed at the maximum transverse distance of any other point along the length of the strut. In other embodiments, the riser element 673 may be formed other than by a bend in the strut 655a-d, such as by structure added to the strut or by another suitable technique.

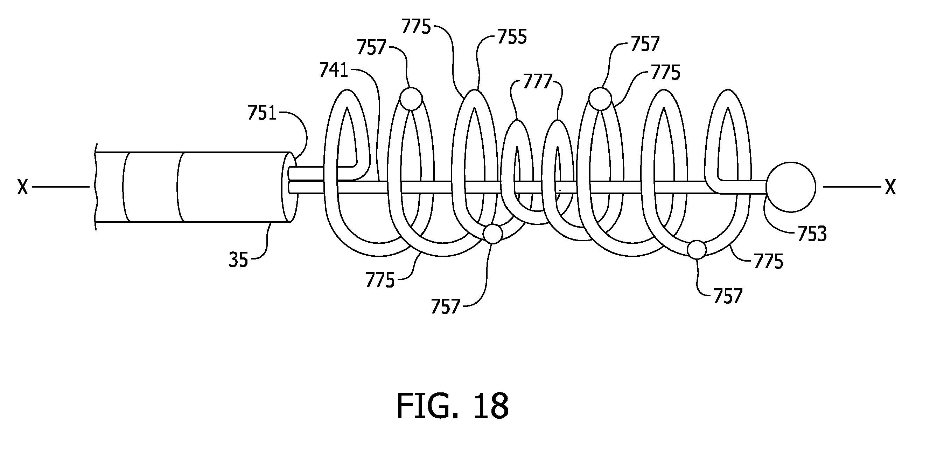

Figure 18 illustrates another suitable embodiment of an electrode assembly 733 for use with the catheter system 21 illustrated in Figures 1 and 2. The electrode assembly 733 is configurable between a collapsed configuration for maneuvering and positioning the electrode assembly in the patient, and an expanded configuration (not shown) for operation of the electrode assembly to perform a desired procedure such as an ablation procedure. As seen in Figure 18, a control line, such as a suitable cable or pull wire 741, extends within the hollow catheter shaft 29 and into the handle 25 for operative connection with the actuator 37 to thereby operatively connect the actuator 37 with the electrode assembly.

The illustrated electrode assembly 733 comprises a single (e.g., unitary) coil 755 extending from a proximal end 751 to a distal end 753 of the electrode assembly about a longitudinal axis X of the electrode assembly. The coil 755 carries at least one electrode 757 disposed at a respective longitudinal position along the coil, i.e., at a respective longitudinal distance along the longitudinal axis X from the proximal end of the electrode assembly. In the embodiment of Figure 18, a plurality of electrodes 757 are spaced along the length of the coil 755 at different longitudinal positions. It is understood, however, the electrodes 757can be disposed at any suitable position along the coil 755. It is also understood that the coil 755 can carry any suitable number of electrodes 757.

The electrode assembly 733 has a length defined by the extent of the control line 741 along the longitudinal axis X from the proximal end 751 to the distal end 753 of the electrode assembly. To configure the electrode assembly 733 from its collapsed configuration to its expanded configuration, rotation of the actuator 37 (as indicated by the arrow in Figure 2) relative to the handle 25 operatively pulls on the control wire 741 to thereby pull the distal end 753 of the electrode assembly toward the proximal end 751 of the electrode assembly along the longitudinal axis X thereof. As the distance between the distal end 753 and the proximal end 751 of the electrode assembly 733 is shortened (i.e., as the length of the electrode assembly decreases), the coil 755 is longitudinally compressed such that the coil expands in radius and forced outward away from the longitudinal axis X of the electrode assembly to form the expanded configuration of the electrode assembly.

In the illustrated embodiment of Figure 18, the coil 755 is constructed of a single coil having a plurality of winds. More particularly, the coil 755 includes one or more first winds 775 having a first radius and on which the electrodes are disposed, and one or more second winds 777 having a second radius that is substantially less than the radius of the first winds. For example, in one embodiment the first radius may be about 10 mm while the second radius may be about 4-6 mm. The radius differential facilitates apposition of the larger radius winds 775 against the arterial wall without possible interference by the smaller radius winds 777.

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense.

## Claims

1. An electrode assembly for an electrode catheter system, the electrode assembly having a longitudinal axis, a proximal end and a distal end, the electrode assembly comprising:
a plurality of struts each extending from the proximal end to the distal end of the electrode assembly, each strut at least in part spiraling about the longitudinal axis of the electrode assembly intermediate the proximal end and the distal end of the electrode assembly and having a corresponding electrode disposed thereon intermediate said proximal and distal ends of the electrode assembly, wherein the electrode assembly is configurable between a collapsed configuration and an expanded configuration, the electrodes being transversely spaced from the longitudinal axis of the electrode assembly a greater distance in the expanded configuration than in the collapsed configuration, in the expanded configuration the electrodes being transversely equally spaced from the longitudinal axis of the electrode assembly.

2. The electrode assembly of claim 1 wherein each strut at least in part spirals about the longitudinal axis of the electrode assembly through a circumferential rotation angle of about 90 degrees.

3. The electrode assembly of claim 1 wherein each strut comprises a longitundinally extending proximal leg, a longitudinally extending distal leg, and a center segment extending between and interconnecting the proximal leg and the distal leg, the center segment extending at an angle relative to the proximal and distal legs in the range of about 165 degrees to about 175 degrees.

4. The electrode assembly of claim 1 or 2 wherein each electrode is located on its respective strut at a longitudinal distance from the proximal end of the electrode assembly, at least one electrode being at a longitudinal distance different from a longitudinal distance of at least one other electrode.

5. The electrode assembly of any one of claims 1 to 3 wherein the electrode assembly has a length from its proximal end to its distal end, the length of the electrode assembly decreasing upon configuration of the electrode assembly from its collapsed configuration to its expanded configuration.

6. An electrode assembly for an electrode catheter system, the electrode assembly having a longitudinal axis, a proximal end and a distal end, the electrode assembly comprising:
a plurality of struts each extending from the proximal end to the distal end of the electrode assembly, each strut at least in part spiraling about the longitudinal axis of the electrode assembly intermediate the proximal end and the distal end of the electrode assembly and having a corresponding electrode disposed thereon intermediate said proximal and distal ends of the electrode assembly, wherein the electrode assembly is configurable between a collapsed configuration and an expanded configuration, each electrode being spaced transversely from the longitudinal axis of the electrode assembly a greater distance in the expanded configuration than in the collapsed configuration, the electrode assembly including at least one asymmetric element configured to facilitate a symmetrical expansion of the electrode assembly about the longitudinal axis thereof from the collapsed configuration to the expanded configuration of the electrode assembly.

7. The electrode assembly of claim 6 wherein each electrode is disposed on a respective strut at a longitudinal distance from the proximal end of the electrode assembly, the longitudinal distance of one electrode being different from the longitudinal distance of at least one other electrode, in the expanded configuration of the electrode assembly the electrodes being transversely spaced from the longitudinal axis of the electrode assembly approximately the same distance.

8. The electrode assembly of claim 6 or 7 wherein the electrode assembly has a length from its proximal end to its distal end, the length of the electrode assembly decreasing upon configuration of the electrode assembly from its collapsed configuration to its expanded configuration.

9. The electrode assembly of claim 6 or 7 in combination with the catheter system, the catheter system comprising a handle, an elongate shaft extending from the handle, the electrode assembly, and an actuator associated with the handle and operatively connected to the electrode assembly for selectively configuring the electrode assembly from its collapsed configuration to its expanded configuration.

10. An electrode assembly for an electrode catheter system, the electrode assembly having a longitudinal axis, a proximal end and a distal end, the electrode assembly comprising:
a plurality of struts each extending from the proximal end to the distal end of the electrode assembly, each strut having a riser element disposed thereon intermediate the proximal end and the distal end of the electrode assembly, the electrode assembly being configurable between a collapsed configuration and an expanded configuration, in the expanded configuration the riser element of each strut extending transversely outward from the longitudinal axis of the electrode assembly a greater distance than any other point along the respective strut, each strut further having an electrode disposed on the respective riser element.

11. The electrode assembly set forth in claim 10 wherein for each strut the respective riser element comprises a bent segment intermediate the proximal end and the distal end of the electrode assembly, the bent segment having an apex defining the transversely outermost extent of the strut in the expanded configuration of the electrode assembly.

12. The electrode assembly set forth in claim 10 or 11 wherein each riser element is located a longitudinal distance from the proximal end of the electrode assembly, the riser element of one strut being at a longitudinal distance that is different from the longitudinal distance of the riser element of at least one other strut.

13. An electrode assembly for an electrode catheter system, the electrode assembly having a longitudinal axis, a proximal end and a distal end, the electrode assembly comprising:
a unitary coil having a plurality of winds and extending continuously from the proximal end to the distal end of the electrode assembly, and a plurality of electrodes on the coil, each electrode being disposed on a different one of the winds of the coil, the coil being configurable between a collapsed configuration and an expanded configuration, the winds having a radius wherein the radius of the winds increases upon configuration of the coil from the collapsed configuration to the expanded configuration.

14. The electrode assembly of claim 13 wherein the plurality of winds comprises a plurality of first winds each having a first radius and at least one second wind having a second radius less than the first radius, each electrode being disposed on a respective one of the plurality of first winds, in the expanded configuration the first radius of the plurality of first winds being greater than in the collapsed configuration of the coil.

15. The electrode assembly of claim 13 or 14 wherein the coil has a length from its proximal end to its distal end, the length of the coil decreasing upon configuration of the coil from its collapsed configuration to its expanded configuration.
